# EUROPÄISCHE PATENTANMELDUNG

(11) **EP 1 639 974 A1**
(43) Veröffentlichungstag der Anmeldung: **29.03.2006**
(21) Anmeldenummer: 05012171.4
(22) Anmeldetag: 04.06.2005
(51) Int. Cl.: A61F 13/15, A61F 13/20, A61F 13/26

(54) **Tampon mit Umhüllung**

(30) Priorität: 28.09.2004 DE 102004046969
(71) Anmelder: HYSALMA Hygiene Sales & Marketing GmbH, 45481 Mülheim an der Ruhr (DE)
(72) Erfinder: Schmidt, Lothar, 09569 Oederan (DE)
(74) Vertreter: COHAUSZ DAWIDOWICZ HANNIG & SOZIEN

(57) **Zusammenfassung**

Die Erfindung betrifft einen Tampon mit einer schlauchförmigen Umhüllung, aus deren vorderen, sich öffnenden Ende der Tampon herausdrückbar ist. Beim axialen Herausdrücken des Tampons mit seiner vorderen Spitze aus dem vorderen geöffneten Ende der Umhüllung ist die Umhüllung nach außen derart umstülpbar und wendbar, dass der Öffnungsrand der Umhüllung relativ von der vorderen Spitze in Richtung des hinteren Endes des Tampons bewegbar und der Tampon hierdurch frei gebbar ist.

## Beschreibung

Die Erfindung betrifft einen Hygiene-Tampon für Frauen mit einer schlauchförmigen Umhüllung, aus deren vorderen, sich öffnenden Ende der Tampon herausdrückbar ist.

Es ist bekannt, einen Tampon durch einen Applikator in der Scheide einer Frau einzubringen. Hierbei weist der Applikator ein Rohr auf, das an seinem vorderen Ende verschlossen ist und in dem der Tampon einliegt. Durch einen am hinteren Ende des Rohrs, in dieses hineinreichenden Stößel, kann der Tampon aus dem vorderen Ende des Rohrs herausgedrückt werden, wobei das vordere Ende sich öffnet. Bei der Verwendung dieses bekannten Applikators kann dieser verschmutzen, so dass bei seiner mehrfachen Verwendung es zu einem Übertragen von Keimen kommen kann. Eine Mehrfachverwendung eines solchen Applikators ist daher nicht möglich.

Aufgabe der Erfindung ist es, eine Verpackung der eingangs genannten Art so zu verbessern, dass bei einfacher Herstellung eine hygienische und leicht handhabbare Benutzung gegeben ist.

Diese Aufgabe wird erfindungsgemäß dadurch gelöst, dass beim axialen Herausdrücken des Tampons mit seiner vorderen Spitze aus dem vorderen geöffneten Ende der Umhüllung die Umhüllung nach außen derart umstülpbar und wendbar ist, dass der Öffnungsrand der Umhüllung relativ von der vorderen Spitze in Richtung des hinteren Endes des Tampons bewegbar und der Tampon hierdurch frei gebbar ist.

Bei einem solchen Tampon schützt die sich nach außen umstülpbare Umhüllung das Mittel, durch das der Tampon aus der Umhüllung herausgedrückt wird. Dies kann zum einen ein Finger und zum anderen ein Applikator sein. In beiden Fällen sorgt die Umhüllung dafür, dass der Finger oder der Applikator mit der unteren Körperregion nicht in Berührung kommt, so dass eine äußerst hygienische Benutzung möglich ist. Hierbei ist von Bedeutung, dass die erfindungsgemäße Umhüllung als Verpackung und/oder als Schutz für den Finger oder für den Applikator dient. Die Herstellung ist besonders einfach und die Verwendung unkompliziert und selbst erklärend.

Der Tampon kann durch Druck eines Fingers auf das rückseitige Ende des Tampons aus der Umhüllung herausgedrückt werden, ohne einen Applikator zu benötigen. Hierbei stülpt sich die Umhüllung über den Finger, so dass dieser keimfrei bleibt.

Ein besonders sicherer Halt der Umhüllung während des Einschiebens des Tampons ist dann gegeben, wenn die Umhüllung an ihrem vorderen Ende eine insbesondere ringförmige Erweiterung bildet, die von einer Hand insbesondere deren Fingern hintergreifbar ist.

Alternativ wird vorgeschlagen, dass der Tampon mit seiner schlauchförmigen Umhüllung in einen mehrfach benutzbaren Applikator einlegbar ist und durch einen Stößel des Applikators aus der Umhüllung herausdrückbar ist. Auch hierbei ist sichergestellt, dass der Applikator hygienisch sauber bleibt, da die schlauchförmige Umhüllung sich beim Herausdrücken auf die Außenseite des Applikators legt.

Beim Herausdrücken des Tampons aus der Umhüllung öffnet sich das vordere Ende und stülpt sich um, so dass die Umhüllung sich über sich selbst schiebt. Hierbei bleibt die Umhüllung vorzugsweise aufgrund ihres elastischen Materials unbeschädigt, das heißt bis auf die vordere Öffnung erfolgt kein Einreißen der Umhüllung.

Zwei Ausführungsbeispiele der Erfindung sind in den Zeichnungen dargestellt und werden im folgenden näher beschrieben. Es zeigen
- Fig. 1: einen Schnitt durch die Umhüllung mit einliegendem Tampon und einem Rückholfaden in einer Tasche,
- Fig. 2: einen Schnitt durch die Umhüllung mit einliegendem Tampon und einem Rückholfaden befestigt am Boden;
- Fig. 3: eine perspektivische Ansicht der Umhüllung nach Aufstellen der umlaufenden Tasche zu einem äußeren Ringwulst;
- Fig. 4: eine perspektivische Ansicht der Umhüllung gehalten in einer Hand;
- Fig. 5: die Umhüllung mit Tampon nach ihrem Aufsetzen auf den Körper;
- Fig. 6: die Umhüllung mit Tampon während des Herausdrückens des Tampons;
- Fig. 7: die Lage von Umhüllung und Tampon nachdem der Tampon die Umhüllung verlassen hat;
- Fig. 8: Umhüllung und Tampon zu Beginn der Entfernung des an der Umhüllung über einen Faden befestigten Tampons.

Der längliche, an seinem vorderen Ende abgerundete Tampon 1 aus Flüssigkeit aufsaugendem Material ist vor seiner Benutzung von einer, vorzugsweise zylindrischen Umhüllung 2 über seine gesamte Länge umgeben, so dass der Tampon in der eine Verpackung bildenden Umhüllung 2 vollständig und geschützt einliegt. Am vorderen Ende weist die Umhüllung 2 eine Sollbruchstelle in Form von sich kreuzenden Schwächungslinien 4 auf, so dass dort beim Herausschieben des Tampons sich eine Öffnung bildet, durch die der Tampon herausschiebbar ist.

Die Umhüllung 2 besteht aus einem solch elastischen Material, dass beim Einführen des Tampons in die Scheide einer Frau die Umhüllung 2 sich nach außen umstülpt und damit der Öffnungsrand 3 der Umhüllung sich vom vorderen Ende aus nach hinten über die Umhüllung schiebt, wodurch die Umhüllung gewendet wird.

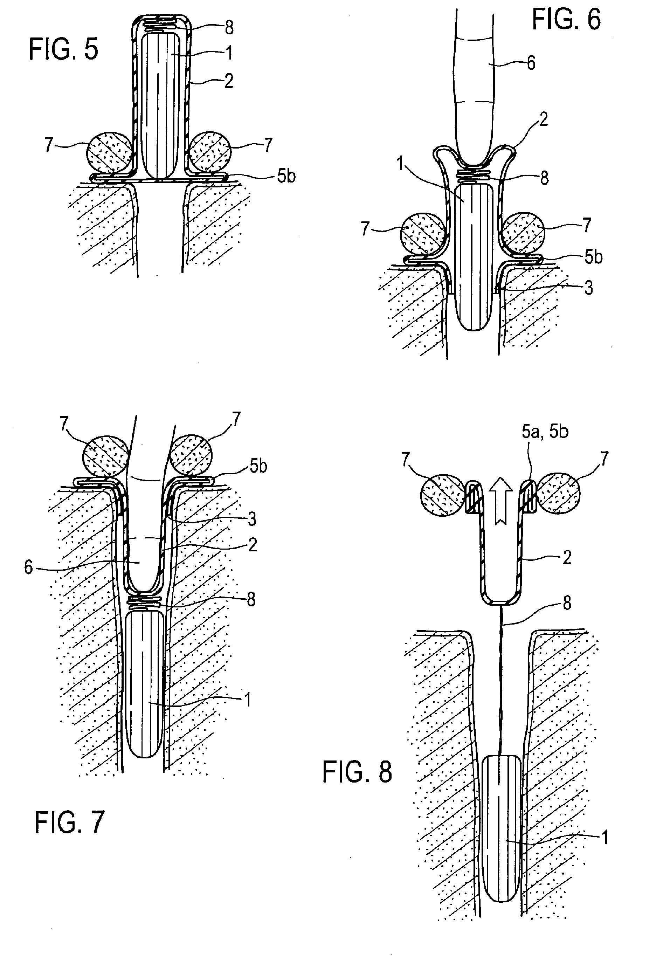

Wird der Tampon 1 durch einen Finger 6 aus der Umhüllung 2 herausgedrückt, indem der Finger auf das rückseitige Ende des Tampons und der Umhüllung drückt, so schiebt sich die Umhüllung über den Finger 6, wie dies in Fig. 6 und 7 dargestellt ist. Damit bildet die Umhüllung 2 einen Schutz für den Finger 6. Während des Umstülpens über den Finger ist es von Vorteil, wenn die Umhüllung gegen den Körper fixiert wird oder mit zwei Fingern 7 der anderen Hand festgehalten wird.

Dieses Festhalten wird erleichtert, wenn die Umhüllung 2 an ihrem dem Körper zugewandten vorderen Ende eine umlaufende ringförmige Erweiterung bildet, die nach außen bewegbar und erweiterbar ist, um dann einen taschenförmigen Ringwulst 5b zu bilden, der als Flansch auf den Körper auflegbar ist, an seiner Vorderseite die Schwächungslinie bildet und von zwei Fingern 7 hintergreifbar ist.

Die erfindungsgemäße Verpackung kann auch in einem nicht dargestellten Applikator verwendet werden. Hierbei besitzt der Applikator ein zylindrisches, nach vorne offenes Rohr, in den der Tampon mit seiner Verpackung einschiebbar ist. In das rückseitige Ende des Rohrs ragt ein Stift hinein, der in das Rohr eingedrückt wird, wodurch der Tampon 1 aus der Umhüllung 2 nach vorne herausgedrückt wird, wie bereits oben beschrieben. Hierbei wird die Umhüllung 2 über die Außenseite des zylindrischen Rohrs gestülpt und gewendet, so dass die Außenseite hierdurch geschützt wird.

Am hinteren Ende des Tampons ist ein Faden 8 befestigt, um ein Herausziehen des Tampons aus der Scheide zu erleichtern. Statt eines Fadens kann aber auch die Umhüllung 2 am hinteren Ende des Tampons 1 befestigt sein, um damit die Aufgabe des Fadens zu übernehmen. Dabei kann die Umhüllung 2 auch über den Rückholfaden 8 am Tampon 1 befestigt sein.

Die Handhabung des Tampons mit Umhüllung geschieht von Hand wie folgt:

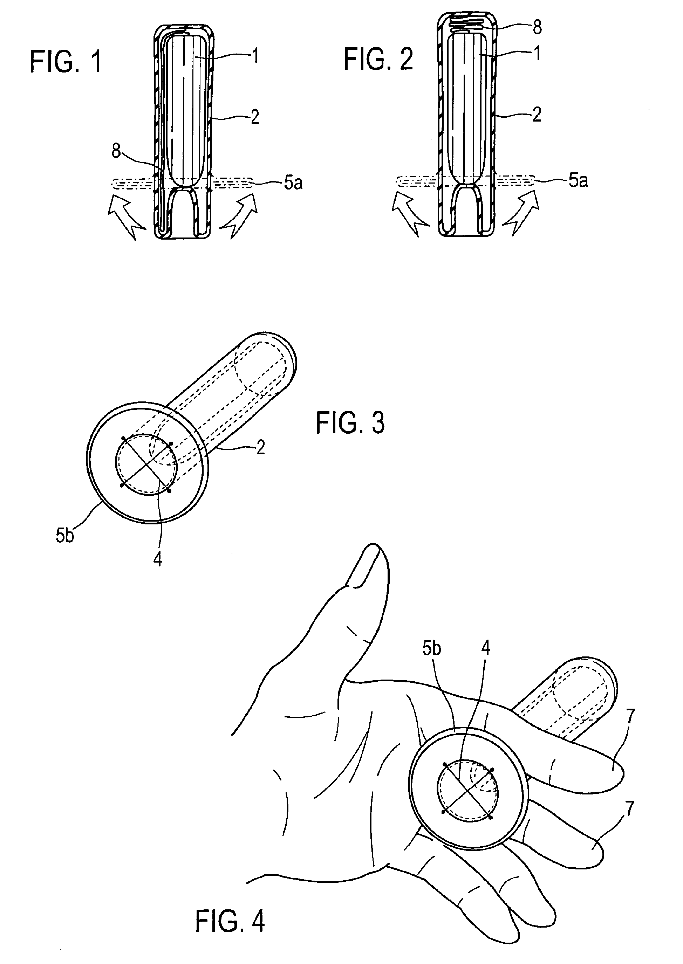

Fig. 4 zeigt die Aufnahme eines Tampons innerhalb seiner Verpackung mit ungebördeltem Wulst, so wie er in der Hand der Anwenderin zu liegen kommt, bevor der Tampon appliziert wird.

In dieser oder in einer ähnlichen Anordnung wird der Tampon mit dem Wulst 5 und zum Körper gerichteter Öffnungshilfe/Sollbruchstelle an die Vagina der Benutzerin angelegt.

Die Finger pressen den Wulst der Tamponverpackung gegen die Vagina und halten somit die Verpackung mit dem darin befindlichen Tampon in Position vor dem Gebärmutterkanal.

Fig. 6 zeigt den Beginn des Einschubvorgangs des Tampons in den Gebärmutterkanal. Die Tamponverpackung 2 wird durch die anpressenden Finger in ihrer Lage fixiert. Mit einem Finger der zweiten Hand wird von außen Druck auf die Verpackung mit dem darin befindlichen Tampon ausgeübt. Dieser Druck pflanzt sich über den innen liegenden Tampon auf die mit Schwächungslinien versehene Bodenfläche fort. In der Folge dieser Druckausübung platzt die Tamponverpackung gezielt an den präparierten Sollbruchstellen. Die Tamponverpackung wölbt sich nun blütenartig in den Gebärmutterkanal hinein. Der Tampon kann mit dem Finger in den Gebärmutterkanal hineingeschoben werden. Die Verpackung selbst bildet nun auf der Fingerseite einen Kanal, der den Finger selbst vor dem Kontakt mit den Schleimhäuten der Vagina und somit vor Verschmutzung schützt.

Fig. 7 zeigt den vollständig in seine Endposition eingeschobenen Tampon. Der einschiebende Finger ist weitgehend in den Gebärmutterkanal eingetaucht. Der aus der Tamponverpackung gebildete Schlauch schützt den Finger vor Verschmutzung.

Fig. 8 zeigt die Beendigung des Applikationsvorgangs. Die nun leere Tamponverpackung wurde aus dem Gebärmutterkanal entfernt (herausgezogen).

Dabei hat sich der spiralförmig am Tamponbodenende anliegende Rückholfaden gestreckt. Das Ende des Rückholfadens mit dem Sicherheitsknoten wurde aus dem Gebärmutterkanal herausgezogen. Das nur leicht befestigte Ende der Rückholschnur an der Tamponverpackung löst sich bei weiterer Ausübung von Zugkraft selbst ab und verbleibt bestimmungsgemäß außerhalb des Körpers der Benutzerin.

Die Skizzen und die vorstehende Beschreibung bezieht sich auf die Tamponverpackungsausführung nach Fig. 2. Bei der Ausführung nach Fig. 1 ändert sich lediglich der in Fig. 8 beschriebene Vorgang. Dabei wird das in die Wulsttasche eingelegte Rückholfadenende erst gar nicht in den Gebärmutterkanal eingeführt, sondern verbleibt, von den Fingern zusätzlich fixiert, außerhalb des Körpers.

## Patentansprüche

1. Tampon (1) mit einer schlauchförmigen Umhüllung (2), aus deren vorderen, sich öffnenden Ende der Tampon herausdrückbar ist, **dadurch gekennzeichnet, dass** beim axialen Herausdrücken des Tampons (1) mit seiner vorderen Spitze aus dem vorderen geöffneten Ende der Umhüllung die Umhüllung (2) nach außen derart umstülpbar und wendbar ist, dass der Öffnungsrand (3) der Umhüllung relativ von der vorderen Spitze in Richtung des hinteren Endes des Tampons bewegbar und der Tampon hierdurch frei gebbar ist.

2. Tampon nach Anspruch 1,**dadurch gekennzeichnet, dass** der Tampon (1) durch Druck eines Fingers (6) auf das rückseitige Ende des Tampons aus der Umhüllung (2) drückbar ist.

3. Tampon nach Anspruch 2, **dadurch gekennzeichnet , dass** beim Herausdrücken die Umhüllung sich über den Finger (6) schiebt.

4. Tampon nach einem der vorherigen Ansprüche, **dadurch gekennzeichnet, dass** die Umhüllung an ihrem vorderen Ende eine insbesondere ringförmige Erweiterung (5a) bildet, die von einer Hand insbesondere deren Fingern (7) hintergreifbar ist.

5. Tampon nach Anspruch 1, **dadurch gekennzeichnet , dass** der Tampon (1) mit seiner schlauchförmigen Umhüllung (2) in einen mehrfach benutzbaren Applikator einlegbar ist und durch einen Stößel des Applikators aus der Umhüllung (2) herausdrückbar ist.

6. Tampon nach Anspruch 5, **dadurch gekennzeichnet, dass** die schlauchförmige Umhüllung (2) sich beim Herausdrücken auf die Außenseite des Applikators legt.

7. Tampon nach einem der vorherigen Ansprüche, **dadurch gekennzeichnet, dass** die Umhüllung (2) am hinteren Ende des Tampons (1) befestigt ist.

8. Tampon nach einem der vorherigen Ansprüche, **dadurch gekennzeichnet, dass** die Umhüllung (2) am Rückholfaden (8) des Tampons (1) befestigt ist.
